# EUROPEAN PATENT SPECIFICATION

(11) **EP 3 421 034 B1**
(45) Date of publication and mention of the grant of the patent: **16.11.2022**
(21) Application number: 17775932.1
(22) Date of filing: 31.03.2017
(51) Int. Cl.: A61K 9/50, A61K 9/20, A61K 31/18, A61K 31/4985, A61K 9/16, A61K 9/28, A61K 9/48, A61P 9/12, A61P 13/08, A61P 43/00, A61P 9/00

(54) **COMPOSITE CAPSULE PREPARATION CONTAINING TADALAFIL AND TAMSULOSIN AND HAVING IMPROVED STABILITY AND ELUTION RATE**
ZUSAMMENGESETZTES KAPSELPRÄPARAT MIT TADALAFIL UND TAMSULOSIN UND MIT VERBESSERTER STABILITÄT UND ELUTIONSRATE
PRÉPARATION D'UNE CAPSULE COMPOSITE CONTENANT DU TADALAFIL ET DE LA TAMSULOSINE ET AYANT UNE STABILITÉ ET UNE VITESSE D'ÉLUTION AMÉLIORÉES

(30) Priority: 31.03.2016 KR 20160039687
(43) Date of publication of application: 02.01.2019
(73) Proprietor: Hanmi Pharm. Co., Ltd., Hwaseong-si, Gyeonggi-do 18536 (KR)
(72) Inventor: TAK, Jin Wook, Hwaseong-si Gyeonggi-do 18598 (KR); CHO, Jung Hyun, Hwaseong-si Gyeonggi-do 18396 (KR); KIM, Jin Cheul, Seoul 07216 (KR); KIM, Yong Il, Suwon-si Gyeonggi-do 16325 (KR); PARK, Jae Hyun, Suwon-si Gyeonggi-do 16707 (KR); WOO, Jong Soo, Suwon-si Gyeonggi-do 16420 (KR)
(74) Representative: Marks & Clerk LLP
(86) International application number: PCT/KR2017/003603
(87) International publication number: WO 2017/171508

(56) References cited:
- WO-A1-2014/209087
- KR-A- 20080 007 252
- KR-A- 20130 039 797
- KR-A- 20150 002 550
- KR-A- 20150 056 444
- KR-A- 20160 002 411
- KR-A- 20160 002 412

## Description

### TECHNICAL FIELD

The present disclosure relates to a capsule composite formulation including tadalafil and tamsulosin, and more particularly, to a capsule composite formulation including tadalafil and tamsulosin, which may exhibit sufficient dissolution rates along with improved stabilities of tadalafil and tamsulosin.

### BACKGROUND ART

Tadalafil is a substance belonging to the phosphodiesterase 5 (PDE 5) inhibitors such as sildenafil and vardenafil. Tadalafil has a half-life 3 times or longer than that of sildenafil or vardenafil. Tadalafil was originally developed by ICOS Corporation. Cialis^{™}, which is a treatment for erectile dysfunction containing tadalafil, and Adcirca^{™}, which is a treatment for pulmonary arterial hypertension, are currently offered on the market by Eli Lilly and Company. Cialis was approved in 2011 by the FDA as a treatment for benign prostatic hyperplasia. In addition, sildenafil can only be administered when necessary, and thus its safety has not been proven for patients who take it daily for treatment of prostatic hyperplasia. In contrast, tadalafil (Cialis) can be administered once daily in a dose of 5 mg, and therefore, it may be suitably taken daily for the treatment of prostatic hyperplasia or taken together with another therapeutic agent for prostatic hyperplasia which is taken daily.

Tamsulosin is an α1a blocker that is effective in the treatment of symptoms of benign prostatic hyperplasia, chronic prostatitis, and chronic abdominal pain. In addition, by blocking α1a, tamsulosin is also effective in the treatment of urolithiasis via a skeletal muscle relaxation mechanism. Tamsulosin was developed by Yamanouchi Phannaceutical Co., Ltd. in 1996, and various products containing tamsulosin hydrochloride are known.

Erectile dysfunction and benign prostatic hyperplasia may occur alone, independent of each other. However, erectile dysfunction and benign prostatic hyperplasia are likely to occur in the same patient, and according to a study, 8.5 patients out of 10 erectile dysfunction patients in Korea also had prostate gland diseases. Accordingly, there is a need for the development of a therapeutic method of treating the two diseases simultaneously with excellent stability and efficacy. In particular, although the mechanism of action of tadalafil differs from that of tamsulosin, they are both effective in the treatment of erectile dysfunction and benign prostatic hyperplasia. Therefore, more excellent effects in terms of prevention and treatment of erectile dysfunction and benign prostatic hyperplasia may be obtained by administering both tadalafil and tamsulosin simultaneously or at intervals as a combined therapy, and adverse effects due to long-term administration may be alleviated by reducing the dosage of each individual drug.

Patent document 1 discloses a composite formulation for oral administration, in which drug compliance of patients is improved by including tadalafil and tamsulosin in one formulation. The composite formulation is a capsule composite formulation, in which tadalafil and tamsulosin are each formulated as an independent part in the form of a granule, a tablet, or a combination thereof, and then included in one capsule. The capsule composite formulation enables separation of the two active ingredients in the capsule, and thus minimizes reactivity between the active ingredients without influencing their dissolution rates, thereby providing excellent stability and dissolution rates.

However, although tadalafil and tamsulosin are included in the capsule of the capsule composite formulation after being respectively formulated as independent solid-phase parts, interaction between tadalafil and tamsulosin is not completely blocked, and it is still necessary to develop a composite formulation in which the active ingredients have excellent stabilities and dissolution rates.

### [Prior Art Documents]

### [Patent Document]

1. WO 2014/209087

### DESCRIPTION OF EMBODIMENTS

### TECHNICAL PROBLEM

An aspect of the present disclosure provides a capsule composite formulation including both tadalafil and tamsulosin, in which active ingredients have excellent stabilities and dissolution rates.

Another aspect of the present disclosure provides a method of preparing the capsule composite formulation.

### SOLUTION TO PROBLEM

An aspect of the present disclosure provides a capsule composite formulation including, in a separated state, an independent tadalafil part including tadalafil or a pharmaceutically acceptable salt thereof; and an independent tamsulosin part including tamsulosin or a pharmaceutically acceptable salt thereof, wherein the independent tadalafil part includes, on a surface thereof, a film coating layer including polyvinyl alcohol (PVA) or a PVA-containing copolymer as a coating base material, wherein the PVA or the PVA-containing copolymer is comprised in an amount of 1 % by weight to 6% by weight with respect to the total weight of the independent tadalafil part excluding the coating layer, and wherein the independent tadalafil part is a tablet of compressed granules, and the granules comprise 10% by weight to 60% by weight of granules having a granule size of 250 µm to 500 µm and 30% by weight or more of granules having a granule size of 150 µm or less.

Another aspect of the present disclosure provides a method of preparing the capsule composite formulation according to an aspect of the present disclosure, the method including:
preparing an independent tadalafil part by mixing tadalafil or a pharmaceutically acceptable salt thereof with a pharmaceutically acceptable additive to prepare tadalafil tablet prepared from the granules;
using a sieve or an oscillator in order to obtain the tadalafil granules having an appropriate granule size;
coating the prepared tadalafil tablet with a coating base material including PVA or a PVA-containing copolymer;
preparing an independent tamsulosin part by mixing tamsulosin or a pharmaceutically acceptable salt thereof with a pharmaceutically acceptable additive to prepare tamsulosin granules or a pellet or a tablet prepared from the granules; and
encapsulating a hard capsule with the prepared coated tadalafil tablet and the prepared tamsulosin granule, pellet, or tablet, in a separated state.

### ADVANTAGEOUS EFFECTS OF DISCLOSURE

Since a capsule composite formulation according to an aspect of the present disclosure includes both tadalafil and tamsulosin in one unit formulation while including a film coating layer including polyvinyl alcohol (PVA) or PVA-containing copolymer as a coating base material on a surface of an independent tadalafil part, interaction between tadalafil and tamsulosin may be effectively blocked, thereby remarkably improving stability of each of tadalafil and tamsulosin, and an initial dissolution rate of tadalafil may not be inhibited below a reference dissolution rate, thereby showing no significant impact on efficacy thereof. Accordingly, an aspect of the present disclosure may provide the capsule composite formulation including both tadalafil and tamsulosin, wherein both the active ingredients have excellent stability and dissolution rates.

### BRIEF DESCRIPTION OF DRAWINGS

FIG. 1 is a graph showing results of measuring total related compounds of tamsulosin hydrochloride at an initial point, 1 week, 2 weeks, and 4 weeks after storing capsule composite formulations of Examples 1 to 8 under stress conditions (60°C);
FIG. 2 is a graph showing results of measuring total related compounds of tamsulosin hydrochloride at an initial point, 1 week, 2 weeks, and 4 weeks after storing capsule composite formulations of Comparative Examples 1 to 10 and Examples 9 and 10 under stress conditions (60°C);
FIG. 3 is a graph showing results of measuring total related compounds of tamsulosin hydrochloride at an initial point, 1 month, 3 months, and 6 months after storing capsule composite formulations of Examples 1 to 8 under accelerated conditions (40°C/75% RH);
FIG. 4 is a graph showing results of measuring total related compounds of tamsulosin hydrochloride at an initial point, 1 month, 3 months, and 6 months after storing capsule composite formulations of Comparative Examples 1 to 10 and Examples 9 and 10 under accelerated conditions (40°C/75% RH);
FIG. 5 is a graph showing results of measuring 10 min dissolution rates of tadalafil at an initial point, 1 month, 3 months, and 6 months after storing capsule composite formulations of Examples 4 and 8 and Comparative Examples 4, 7, and 10 under accelerated conditions (40°C/75% RH);
FIG. 6 is a graph showing results of measuring 30 min dissolution rates of tadalafil at an initial point, 1 month, 3 months, and 6 months after storing capsule composite formulations of Examples 4 and 8 and Comparative Examples 4, 7, and 10 under accelerated conditions (40°C/75% RH);
FIG. 7 is a graph showing initial tadalafil dissolution profiles of Examples 1 to 4 and Examples 9 and 10;
FIG. 8 is a graph showing particle size distributions of tadalafil granules of Examples 4, 11, and 12;
FIG. 9 shows photographs showing appearances of tadalafil tablets of Examples 4, 11, and 12 after tabletting; and
FIG. 10 is a graph showing results of dissolution rate measurements according to particle sizes of tadalafil granules of Examples 4, 11, and 12.

### BEST MODE

Hereinafter, the present disclosure will be described in more detail.

The present inventors have developed a composite formulation which is prepared by including two kinds of tadalafil and tamsulosin drugs in one unit formulation, thereby increasing drug compliance of a patient who is in need of administration of two drugs of tadalafil and tamsulosin and securing stabilities and dissolution rates of the respective active ingredients at the same time. As a result, it was found that when an independent tadalafil part is introduced with a film coating layer including polyvinyl alcohol (PVA) or a PVA-containing copolymer during preparation of the composite capsule formulation including the independent tadalafil part and an independent tamsulosin part, storage stabilities of tadalafil and tamsulosin may be secured without adverse effects on dissolution of tadalafil, leading to development of the tadalafil and tamsulosin-containing capsule composite formulation having both stability and efficacy.

Accordingly, an aspect of the present disclosure provides a capsule composite formulation including, in a separated state, an independent tadalafil part as defined in the claims including tadalafil or a pharmaceutically acceptable salt thereof; and an independent tamsulosin part including tamsulosin or a pharmaceutically acceptable salt thereof, wherein the independent tadalafil part includes, on the surface thereof, a film coating layer including PVA or a PVA-containing copolymer as a coating base material.

The PVA or PVA-containing copolymer which is a coating agent used for film coating of a solid formulation such as a granule, a pellet, or a tablet may include any known PVA or PVA-containing copolymer.

The PVA-containing copolymer may be a polyvinyl alcohol-polyethylene glycol copolymer (PVA-PEG copolymer), but is not limited thereto.

A molecular weight of the PVA may be about 13,000 to about 50,000. In a specific embodiment, the molecular weight of the PVA may be about 26,000 to about 30,000.

In a specific embodiment, the PVA may be PVA having a degree of hydrolysis of about 86.5% to about 89.0%, viscosity of about 4.8 mpa.s to about 5.8 mpa.s when prepared in a 4 w/w% aqueous solution at about 20°C, and a pH of about 5.0 to about 6.5. When PVA exceeds the above conditions, it is apprehended that problems of a lowered coating adhesion rate, reduced coating uniformity, and failure of a coating machine may be generated at the time of coating.

In a specific embodiment, the capsule composite formulation may be a capsule composite formulation including, in a separated state, an independent tadalafil part including tadalafil or a pharmaceutically acceptable salt thereof; and an independent tamsulosin part including tamsulosin or a pharmaceutically acceptable salt thereof, wherein the independent tadalafil part includes, on the surface thereof, a film coating layer including PVA or a PVA-containing copolymer as a coating base material, and the PVA is PVA having a molecular weight of 13,000-50,000, a degree of hydrolysis of about 86.5% to about 89.0%, viscosity of about 4.8 mpa.s to about 5.8 mpa.s when prepared in a 4 w/w% aqueous solution at about 20°C, and a pH of about 5.0 to about 6.5.

In a specific embodiment, since the film coating layer including PVA or a PVA-containing copolymer is introduced into the independent tadalafil part, the capsule composite formulation may have remarkably excellent storage stabilities of tadalafil and tamsulosin, as compared with a capsule composite formulation which is packed with an independent tadalafil part having no film coating layer or being coated with other different kind of coating base material (see Experimental Example 1). Further, due to introduction of the film coating layer including PVA or a PVA-containing copolymer, the capsule composite formulation may exhibit remarkably excellent dissolution rates of the active ingredients, as compared with a capsule composite formulation which has an independent tadalafil part being coated with other different kind of coating base material (see Experimental Example 2). In order to exhibit an efficacy and an effect equal to those of a known single formulation, the capsule composite formulation according to an aspect of the present disclosure is required to exhibit a tadalafil dissolution rate equivalent to or higher than a dissolution rate (10 min 40(Q)%, 30 min 80(Q)% or more) of the known single formulation, and it was confirmed that the capsule composite formulation meets criterion of the tadalafil dissolution rate due to introduction of the film coating layer including PVA or a PVA-containing copolymer.

Therefore, it was confirmed that the capsule composite formulation may secure storage stabilities of tadalafil and tamsulosin and excellent dissolution rates thereof at the same time due to introduction of the film coating layer including PVA or a PVA-containing copolymer.

The PVA or PVA-containing copolymer which is a coating base material forms a coating layer on the surface of the independent tadalafil part according to a common film coating method and film coating dose. The PVA or PVA-containing copolymer is comprised in an amount of 1% by weight to 6% by weight with respect to the total weight of the independent tadalafil part (e.g. uncoated tablet) excluding the coating layer. When the amount exceeds the above range, dissolution rates of the active ingredients tend to decrease, which may influence drug efficacy (see Experimental Example 2).

The film coating layer may include any known additive for film coating which is needed to form film coating, in addition to the coating base material. The additive for film coating may include a coloring agent, a coating aid (plasticizer), a flavoring agent (a strawberry flavor, a blueberry flavor, an orange flavor, a peppermint flavor, etc.), a stabilizer (NH₃, sodium lauryl sulfate, etc.), or any combination thereof. The coloring agent may include talc, titanium dioxide (TiO₂), yellow iron oxide, red iron oxide, black iron oxide, brown iron oxide, or blue iron oxide, etc., but is not limited thereto. The coating aid may include polyethylene glycol, propylene glycol, triacetin, ethylcitrate ester, castor oil, or polysorbates, etc., but is not limited thereto.

In a specific embodiment, the film coating layer may include PVA, titanium dioxide, PEG3350, talc, and yellow iron oxide. In another specific embodiment, the film coating layer may include a PVA-PEG copolymer, titanium dioxide, PEG3350, talc, and yellow iron oxide.

The independent tamsulosin part may be coated with a pharmaceutically acceptable coating base material. The coating base material may include, for example, methyl cellulose, ethyl cellulose, polyvinyl acetate, polyvinyl alcohol, polyvinyl pyrrolidone, povidone, a methacrylic acid-ethyl acrylate copolymer, triacetin, propylene glycol, hydroxyethyl cellulose, hydroxypropyl methyl cellulose, etc., but is not limited thereto. In a specific embodiment, the independent tamsulosin part may include, on the surface thereof, an enteric coating layer including an enteric coating base material.

In a specific embodiment, the capsule composite formulation may include the independent tadalafil part including, on the surface thereof, the film coating layer including the PVA or PVA-containing copolymer and the independent tamsulosin part including, on the surface thereof, the enteric coating layer including the enteric coating base material.

The independent tadalafil part is a tablet and the independent tamsulosin part may be in a granule, pellet, or tablet form. When existing as tablets, the independent tadalafil part and the independent tamsulosin part are not intermixed with each other, and they may more completely exist in a separated state. In another specific embodiment, the independent tadalafil part may be a tablet and the independent tamsulosin part may be a pellet.

In a specific embodiment, the independent tadalafil part is a tablet including, on the surface thereof, the film coating layer including the PVA or PVA-containing copolymer, and the independent tamsulosin part may be a pellet including, on the surface thereof, the enteric coating layer including the enteric coating base material. The enteric coating base material may be selected from the group consisting of a methacrylic acid-ethyl acrylate copolymer, triacetin, shellac, cellulose acetate phthalate, hydroxymethylcellulose phthalate, wax, and any combination thereof, but is not limited thereto.

The enteric coating base material may be maintained in a minimal amount for an optimal formulation size and effective preparation. In a specific embodiment, the enteric coating base material may be used in an amount of 0.1% by weight to 20% by weight, or 2% by weight to 10% by weight with respect to the total weight of the independent tamsulosin part.

In the capsule composite formulation, the independent tadalafil part is a tablet of compressed granules, and the granule has 10% by weight to 60% by weight of granules having a granule size of 250 µm to 500 µm (35 mesh ~ 60 mesh) and 30% by weight or more of granules having a granule size of 150 µm (100 mesh) or less. More specifically, in the capsule composite formulation, the granules may include 30% by weight to 50% by weight of granules having a granule size of 250 µm to 500 µm (35 mesh ~ 60 mesh) and 40% by weight or more of granules having a granule size of 150 µm (100 mesh) or less. Here, an average granule size of the tadalafil granule part may be measured by using sieves. The measurement may be performed by a method of vertically stacking sieves of 850 µm, 500 µm, 250 µm, 150 µm, and 75 µm in this order, putting 10 g of granules in the top sieve, shaking the sieves for 5 minutes with a constant speed and vibration, and then measuring a residual amount of the granules in each sieve to calculate the weight ratio. During preparation of the tadalafil granule part, a sieve or oscillator may be used to control the granule size within the above range.

Experimental results showed that when the average granule size is smaller than the above range, an initial dissolution rate of tadalafil meets dissolution criteria, but a capping phenomenon occurs upon tabletting, and when the average granule size is larger than the above range, there are no tabletting restrictions such as a capping phenomenon, etc., but the initial dissolution rate of tadalafil is likely not to meet the dissolution criteria (see Experimental Example 3). Therefore, when granules are prepared for preparation of the tadalafil tablet, the size of the granule may be appropriately controlled within the above range, thereby securing both productivity and initial dissolution rate. In order to exhibit efficacy and effect equal to those of the known tadalafil single formulation, the capsule composite formulation is required to have a tadalafil dissolution rate equivalent to or higher than a dissolution rate (10 min 40(Q)%, 30 min 80(Q)% or more) of the known single formulation. In a specific embodiment, the capsule composite formulation is a capsule composite formulation having a tadalafil dissolution rate of 40(Q)% or more in 10 minutes and a tadalafil dissolution rate of 80(Q)% or more in 30 minutes, according to a dissolution test conducted according to the paddle method of the dissolution test of the United States Pharmacopoeia (USP).

As used herein, the term "tablet of compressed granules" refers to a tablet which is obtained by preparing dry granules or wet granules according to an arbitrary method and then tabletting the granules.

In a specific embodiment, the capsule composite formulation is a capsule composite formulation having total related compounds of 1.0% or less which is a criterion determined with reference to ICH Guidelines, as tested based on the impurity test of Tamsulosin Hydrochloride capsules of USP; and
having a tadalafil dissolution rate of 40(Q)% or more in 10 minutes and a tadalafil dissolution rate of 80(Q)% or more in 30 minutes,, according to a dissolution test conducted according to the paddle method of the dissolution test of the USP.

In a specific embodiment, the capsule composite formulation is a capsule composite formulation including, in a separated state, the independent tadalafil part including tadalafil or a pharmaceutically acceptable salt thereof; and the independent tamsulosin part including tamsulosin or a pharmaceutically acceptable salt thereof, wherein the independent tadalafil part includes, on the surface thereof, the film coating layer including the PVA or PVA-containing copolymer as a coating base material, and the PVA is PVA having a molecular weight of 13,000 to 50,000, and the independent tadalafil part is a tablet of compressed granules, and the granule include 10% by weight ~ 60% by weight of granules having a granule size of 250 µm to 500 µm (35 ~ 60 mesh) and 30% by weight or more of granules having a granule size of 150 µm (100 mesh) or less.

In the capsule composite formulation, the independent tadalafil part and the independent tamsulosin part may include one or more pharmaceutically acceptable additives selected from the group consisting of a diluent, a disintegrant, a binder, a stabilizer, a lubricant, a coloring agent, and any combination thereof, respectively. The diluent, the disintegrant, the binder, the stabilizer, the lubricant, and the coloring agent may be any additive which are known to be commonly used in the art. The diluent may be used in an amount of about 1% by weight to about 95% by weight, and more specifically, about 5% by weight to about 95% by weight with respect to the total weight of each independent part. The binder may be used in an amount of about 0.1% by weight to about 30% by weight, and more specifically, about 2% by weight to about 20% by weight with respect to the total weight of each independent part. The disintegrant may be used in an amount of about 0.1% by weight to about 30% by weight, and more specifically, about 2% by weight to about 15% by weight with respect to the total weight of each independent part. The lubricant may be used in an amount of about 0.3% by weight to about 5% by weight, and more specifically, about 0.5% by weight to about 3% by weight with respect to the total weight of each independent part.

For example, the diluent may be selected from the group consisting of microcrystalline cellulose, lactose, Ludipress, mannitol, calcium dihydrogen phosphate, starch, low-substituted hydroxypropyl cellulose, and mixtures thereof, but is not limited thereto; the binder may be selected from the group consisting of hydroxypropyl cellulose, hydroxypropyl methyl cellulose, hypromellose, polyvinyl acetate, polyvinyl pyrrolidone, copovidone, macrogol, sodium lauryl sulfate, light anhydrous silicic acid, silicate derivatives such as synthetic aluminum silicate, calcium silicate, or magnesium metasilicate aluminate, phosphates such as calcium hydrogen phosphate, carbonates such as calcium carbonate, pregelatinized starch, and gums such as acacia gum, gelatin, cellulose derivatives such as ethyl cellulose, and any combinations thereof, but is not limited thereto; the disintegrant may be selected from the group consisting of crospovidone, sodium starch glycolate, croscarmellose sodium, low-substituted hydroxypropyl cellulose, starch, alginic acid or sodium salts thereof, and any combinations thereof, but is not limited thereto; and the lubricant may be selected from the group consisting of stearic acid, metal salts thereof, talc, colloid silica, sucrose fatty acid ester, hydrogenated vegetable oil, wax, glyceryl fatty acid esters, and any combinations thereof, but is not limited thereto.

The capsule of the capsule composite formulation may be any hard capsule that is commonly used in the art. For example, a hard capsule including hypromellose, pullulan, gelatin, polyvinyl alcohol, or any combination thereof as a capsule base material may be used.

A size of the hard capsule is not limited, as long as the size is a general capsule size used in the preparation of medicinal products. The size of the hard capsule may have a variety of internal capacity depending on the size number of the capsule. For example, it is known that size No. 0 has an internal capacity of about 0.68 mL, size No. 1 about 0.47 mL, size No. 2 about 0.37 mL, size No. 3 about 0.27 mL, and size No. 4 about 0.20 mL. The size of the hard capsule may be as small as possible for the convenience of a patient who takes the capsule composite formulation. However, due to the limitation on the amount of the content to be filled into the capsule, a capsule of size No. 0, 1, 2, 3, or 4 may be used, and a capsule of size No. 1, 2, or 3 may be used.

Examples of the pharmaceutically acceptable salt of tadalafil may include hydrobromide, phosphate, sulfate, hydrochloride, maleate, fumarate, lactate, tartrate, citrate, besylate, camsylate, gluconate, etc. For example, a tadalafil free base may be used, but is not limited thereto.

The capsule composite formulation may include tadalafil or the pharmaceutically acceptable salt thereof in an amount of about 3% by weight to about 7 % by weight with respect to the total weight of the independent tadalafil part. In consideration of a known daily dose, the capsule composite formulation may include about 5 mg to about 20 mg, or about 5 mg to about 10 mg of tadalafil or the pharmaceutically acceptable salt thereof in terms of tadalafil free base, based on a unit formulation.

Examples of the pharmaceutically acceptable salt of tamsulosin may include hydrobromide, phosphate, sulfate, hydrochloride, maleate, fumarate, lactate, tartrate, citrate, besylate, camsylate, gluconate, etc., and a tamsulosin hydrochloride may be used, but is not limited thereto.

The capsule composite formulation may include tamsulosin or the pharmaceutically acceptable salt thereof in an amount of about 0.1% by weight to about 0.4 % by weight with respect to the total weight of the independent tamsulosin part. In consideration of a known daily dose, the capsule composite formulation may include about 0.2 mg to about 0.4 mg of tamsulosin or the pharmaceutically acceptable salt thereof in terms of tamsulosin free base, based on a unit formulation.

The capsule composite formulation may be administered once a day, and taken daily.

The capsule composite formulation may be administered via an administration route including oral route, sublingual route, etc., and it may be orally administered.

Since the capsule composite formulation includes tadalafil or the pharmaceutically acceptable salt thereof and tamsulosin or the pharmaceutically acceptable salt thereof as active ingredients, the capsule composite formulation may be used for the prevention or treatment of erectile dysfunction and benign prostatic hyperplasia.

In particular, the capsule composite formulation may include both tadalafil and tamsulosin in a daily dose in one unit formulation, and therefore, it is possible to prevent or treat both erectile dysfunction and benign prostatic hyperplasia by administrating the capsule composite formulation once a day, thereby remarkably improving drug compliance of a patient who is at risk of both diseases or in need of treatment thereof.

Further, since the capsule composite formulation may include both tadalafil and tamsulosin in one capsule while including the independent tadalafil part including the film coating layer including PVA or PVA-containing copolymer, interaction between tadalafil and tamsulosin may be blocked, thereby securing stabilities of the active ingredients and meeting the dissolution criterion of a tadalafil single formulation at the same time. Therefore, the capsule composite formulation may secure both stabilities and efficacies of the active ingredients while including both tadalafil and tamsulosin in one unit formulation, thereby remarkably improving drug compliance of a patient who is in need of prevention and treatment of both erectile dysfunction and benign prostatic hyperplasia.

Another aspect of the present disclosure provides a method of preparing the capsule composite formulation according to an aspect of the present disclosure, the method including:
preparing the independent tadalafil part by mixing tadalafil or the pharmaceutically acceptable salt thereof with the pharmaceutically acceptable additive to prepare tadalafil tablet prepared from the granules;
providing the independent tadalafil part having an appropriate granule size;
coating the prepared tadalafil tablet with the coating base material including the PVA or PVA-containing copolymer;
preparing the independent tamsulosin part by mixing tamsulosin or the pharmaceutically acceptable salt thereof with a pharmaceutically acceptable additive to prepare tamsulosin granules or a pellet or a tablet prepared from the granules; and
encapsulating a hard capsule with the prepared coated tadalafil tablet and the prepared tamsulosin granule, pellet, or tablet in a separated state.

The description of the capsule composite formulation according to one aspect of the present disclosure may be also applied to a detailed description of the preparation method.

Preparation of the granule, pellet, and tablet may be performed by any method of preparing granules, pellets, and tablets known in the art.

In a specific embodiment, the pellet may be prepared by compression and spheroidization of granules.

Forming of the film coating layer including the PVA or PVA-containing copolymer as the coating base material on the surface of the tadalafil tablet may be performed by a common method of forming the film coating layer. For example, all additives for film coating, including the film coating base material, are mixed in combination to prepare a coating solution, and then the surface of the tableted composite tablet may be coated with the coating solution. A solvent for the preparation of the coating solution may vary depending on a kind, concentration, etc. of the coating base material, and for example, distilled water, ethanol, etc. may be used. The formation of the film coating layer may be performed, for example, by putting the tableted tablet in a pan coater and spraying the coating solution thereto.

### MODE OF DISCLOSURE

Hereinafter, the present disclosure will be described in detail with reference to the following Examples. However, these Examples are for illustrative purposes only, and the scope of the present disclosure is not intended to be limited thereby.

### Examples 1 ∼ 10

Components of the following Table 1, corresponding to an independent tadalafil part, were mixed with each other in a powder form to prepare granules by a wet granulation method, and then the mixture was tableted by a circular punch having a diameter of 5.5 mm. The resulting tadalafil tablets were coated with each of coating solutions which were prepared by dissolving raw materials of respective coating parts described in Examples 1 to 10 of Table 2 in purified water. In this regard, the value of the coating solution of Table 2 was calculated as a ratio to the tadalafil uncoated tablet.

Meanwhile, 0.4 mg of tamsulosin hydrochloride, 21 mg of polyvinyl acetate dispersion, 123.5 mg of microcrystalline cellulose, and 5.5 mg of hypromellose, which are described in the independent tamsulosin part of Table 1, were mixed with each other in a powder form to prepare granules. Subsequently, the tamsulosin granules were coated with an inner coating solution which was prepared by dissolving 0.36 mg of povidone, 0.27 mg of propylene glycol, and 1.84 mg of polyvinyl acetate in purified water, and then further coated with an external coating solution which was prepared by dissolving 2.05 mg of a methacrylic acid-ethyl acrylate copolymer and 0.36 mg of triacetin in water. The coated formulation was finally mixed with 0.2 mg of sucrose stearate.

The tadalafil tablets and the tamsulosin granules thus coated were filled into hard capsule No. 1 having hypromellose as a capsule base material to prepare a capsule composite formulation including 5 mg of tadalafil and 0.4 mg of tamsulosin hydrochloride.

**[Table 1]**

| Independent tadalafil part (unit: mg) | | Independent tamsulosin part (unit: mg) | |
|---|---|---|---|
| Tadalafil | 5.0 | Tamsulosin hydrochloride | 0.4 |
| Mannitol | 54.8 | Polyvinyl acetate | 21.00 |
| Hydroxypropyl cellulose | 3.3 | Microcrystalline cellulose | 123.5 |
| Sodium lauryl sulfate | 0.3 | Polyvinyl acetate | 1.84 |
| Microcrystalline cellulose | 16.1 | Hypromellose | 5.5 |
| Sodium starch glycolate | 4.5 | Povidone | 0.36 |
| Magnesium stearate | 1.2 | Propylene glycol | 0.27 |
| | | Triacetin | 0.36 |
| | | Methacrylic acid-ethyl acrylate copolymer (1:1) 30% | 2.05 |
| | | Sucrose stearate | 0.2 |

**[Table 2]**

| | Coating part (mg) | | | | |
|---|---|---|---|---|---|
| | PVA | TiO₂ | PEG3350 | Talc | Yellow iron oxide |
| Example 1 | 1.02 (1.2%) | 0.61 | 0.52 | 0.38 | 0.02 |
| Example 2 | 2.04 (2.4%) | 1.22 | 1.04 | 0.76 | 0.04 |
| Example 3 | 4.08 (4.8%) | 2.44 | 2.08 | 1.52 | 0.08 |
| Example 4 | 5.10 (6.0%) | 3.05 | 2.60 | 1.90 | 0.10 |
| Example 5 | Kollicoat IR (PVA-PEG copolymer, 75:25%) 1.36 mg (about 1.6% as Kollicoat, 1.2% as PVA) | | | | |
| Example 6 | Kollicoat IR (PVA-PEG copolymer, 75:25%) 2.73 mg (about 3.2% as Kollicoat, 2.4% as PVA) | | | | |
| Example 7 | Kollicoat IR (PVA-PEG copolymer, 75:25%) 5.45 mg (about 6.4% as Kollicoat, 4.8% as PVA) | | | | |
| Example 8 | Kollicoat IR (PVA-PEG copolymer, 75:25%) 6.8 mg (about 8.0% as Kollicoat, 6.0% as PVA) | | | | |

| | PVA | TiO₂ | PEG3350 | Talc | Yellow iron oxide |
|---|---|---|---|---|---|
| Example 9 | 6.80 (8.0%) | 4.07 | 3.47 | 2.53 | 0.13 |
| Example 10 | 8.50 (10.0%) | 5.08 | 4.33 | 3.17 | 0.17 |

Examples 8, 9 and 10 are not in the scope of the claims.

### Comparative Examples 1 to 10 and Examples 11 and 12

Components of Table 1, corresponding to the independent tadalafil part, were mixed with each other in a powder form to prepare granules by a wet granulation method, and then the mixture was tableted by a circular punch having a diameter of 5.5 mm. At this time, Comparative Examples 1 to 10 were sieved by a milling method of the same conditions, and Examples 11 and 12 were sieved by milling methods of different conditions to vary particle sizes thereof. A sieve of 400 µm was used for Comparative Examples 1 to 12, and sieves of 850 µm and 250 µm were used for Examples 11 and 12, respectively. Example 11 included 40% or more of granules having a granule size of 500 µm to 850 µm, and Example 12 included 60% or more of granules having a granule size of 250 µm or less. Thereafter, the resulting tadalafil tablets were coated with each of coating solutions which were prepared by dissolving raw materials of respective coating parts described in Comparative Examples 1 to 10 and Examples 11 and 12 of Table 3 in purified water. In this regard, the value of the coating solution of Table 3 was calculated as a ratio to the tadalafil uncoated tablet.

Meanwhile, 0.4 mg of tamsulosin hydrochloride, 21 mg of polyvinyl acetate dispersion, 123.5 mg of microcrystalline cellulose, and 5.5 mg of hypromellose, which are described in the independent tamsulosin part of Table 1, were mixed with each other in a powder form to prepare granules. Subsequently, the tamsulosin granules were coated with an inner coating solution which was prepared by dissolving 0.36 mg of povidone, 0.27 mg of propylene glycol, and 1.84 mg of polyvinyl acetate in purified water, and then further coated with an external coating solution which was prepared by dissolving 2.05 mg of a methacrylate-ethyl acrylate copolymer and 0.36 mg of triacetin in water. The coated formulation was finally mixed with 0.2 mg of sucrose stearate.

The tadalafil tablets and the tamsulosin granules thus coated were filled into hard capsule No. 1 having hypromellose as a capsule base material to prepare a capsule composite formulation including 5 mg of tadalafil and 0.4 mg of tamsulosin hydrochloride.

**[Table 3]**

| | Coating part (mg) | | | | |
|---|---|---|---|---|---|
| | PVA | TiO₂ | PEG3350 | Talc | Yellow iron oxide |
| Comparative Example 1 | None | | | | |
| Comparative Example 2 | HPMC 2910 1.02 mg (1.2%) | | | | |
| Comparative Example 3 | HPMC 2910 2.04 mg (2.4%) | | | | |
| Comparative Example 4 | HPMC 2910 5.10 mg (6.0%) | | | | |
| Comparative Example 5 | HPC-SSL 1.02 mg (1.2%) | | | | |
| Comparative Example 6 | HPC-SSL 2.04 mg (2.4%) | | | | |
| Comparative Example 7 | HPC-SSL 5.10 mg (6.0%) | | | | |
| Comparative Example 8 | Ethyl cellulose 1.02 mg (1.2%) | | | | |
| Comparative Example 9 | Ethyl cellulose 2.04 mg (2.4%) | | | | |
| Comparative Example 10 | Ethyl cellulose 5.10 mg (6.0%) | | | | |
| Example 11 | 5.10 (6.0%) | 3.05 | 2.60 | 1.90 | 0.10 |
| Example 12 | 5.10 (6.0%) | 3.05 | 2.60 | 1.90 | 0.10 |

### Experimental Example 1: Tamsulosin Stability Test

The capsules of Examples 1 to 10 and Comparative Examples 1 to 10 were stored under stress conditions (60°C) and accelerated conditions (40°C/ 75% RH), and then related compounds thereof were measured at an initial point, 1 week, 2 weeks, and 4 weeks after stress conditions, and at 1 month, 3 months, and 6 months after accelerated conditions, respectively.

4 mg of tamsulosin hydrochloride was put in a 50 ml flask, and 20 ml of a 0.5 N sodium hydroxide solution was added thereto, followed by mixing under shaking at 50°C for 15 minutes. Then, 10 ml of acetonitrile was added thereto, followed by mixing under shaking for 10 minutes. Then, 10 ml of 1 N hydrochloric acid was added thereto, followed by mixing under shanking for 10 minutes. The flask was left to cool and acetonitrile was added up to the marked line. Then, centrifugation was performed at 3500 rpm for 20 minutes, and a supernatant was filtered using a 0.45 µm membrane filter to prepare a sample, which was then subjected to liquid chromatography under the following conditions to measure tamsulosin related compounds.

A standard solution was prepared as follows: about 4 mg of tamsulosin hydrochloride standard was precisely taken and put in a 50 mL volumetric flask, 20 mL of 0.5 mol/L sodium hydroxide solution was added thereto, followed by mixing under shaking at 50°C for 15 minutes. Then, 10 ml of acetonitrile was added thereto, followed by mixing under shaking for 10 minutes. Then, 10 ml of 1 mol/L hydrochloric acid was added thereto, followed by mixing under shanking for 10 minutes. The flask was left to cool and acetonitrile was added up to the marked line. 1 ml of this solution was precisely taken and put in a 100 mL volumetric flask, and a diluent was added up to the marked line. The solution was filtered using a 0.45 µm membrane filter to prepare a standard solution. In this regard, the diluent was used after being prepared by putting 40 mL of 0.5 mol/L sodium hydroxide solution in a 100 mL volumetric flask, adding 20 mL of acetonitrile thereto, followed by mixing under shaking for 10 minutes, and then adding 20 ml of 1 mol/L hydrochloric acid thereto, followed by mixing under shaking for 10 minutes, leaving the flask cool, and then adding acetonitrile up to the marked line.

### <Before main peak>

- Column: a column in which a stainless steel tube having an inner diameter of about 4.6 mm and a length of about 15 cm was packed with an octadecylsilyl silica gel for liquid chromatography having a particle size of about 5 µm or a column equivalent thereto (e.g., Inertsil ODS-2)
- Detector: UV spectrophotometer (measurement wavelength: 225 nm)
- Flow rate: 1.0 ml/min
- Injection volume: 50 µl
- Column temperature: 40°C
- Mobile phase: acetonitrile/buffer solution = 3:7 (the buffer solution was prepared by dissolving 8.7 mL of perchloric acid (70%) and about 3.0 g of NaOH in 1900 mL of purified water, adjusting a pH of the solution to pH 2.0 with 1 N NaOH, and then diluting it with 2000 mL of purified water)

### <After main peak>

- Column: a column in which a stainless steel tube having an inner diameter of about 4.6 mm and a length of about 15 cm was packed with an octadecylsilyl silica gel for liquid chromatography having a particle size of about 5 µm or a column equivalent thereto (e.g., Inertsil ODS-2)
- Detector: UV spectrophotometer (measurement wavelength: 225 nm)
- Flow rate: 1.0 ml/min
- Injection volume: 50 µl
- Column temperature: 40°C
- Mobile phase: acetonitrile/buffer solution = 1:1 (the buffer solution was prepared by dissolving 8.7 mL of perchloric acid (70%) and about 3.0 g of NaOH in 1900 mL of purified water, adjusting a pH of the solution to pH 2.0 with 1 N NaOH, and then diluting it with 2000 mL of purified water)

An acceptable range of the related compounds was determined below 1.0% of a total amount of the related compound before and after a tamsulosin hydrochloride peak.

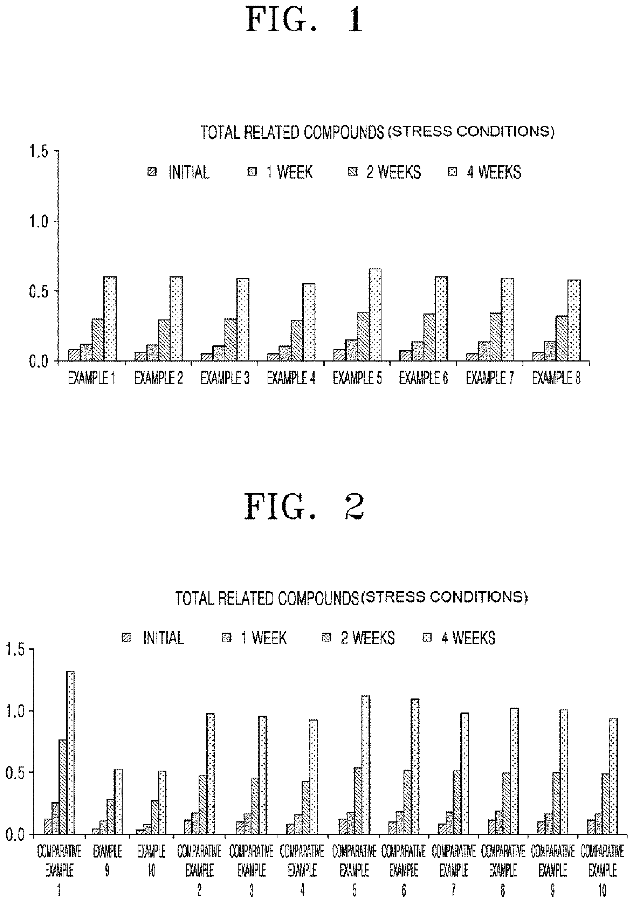

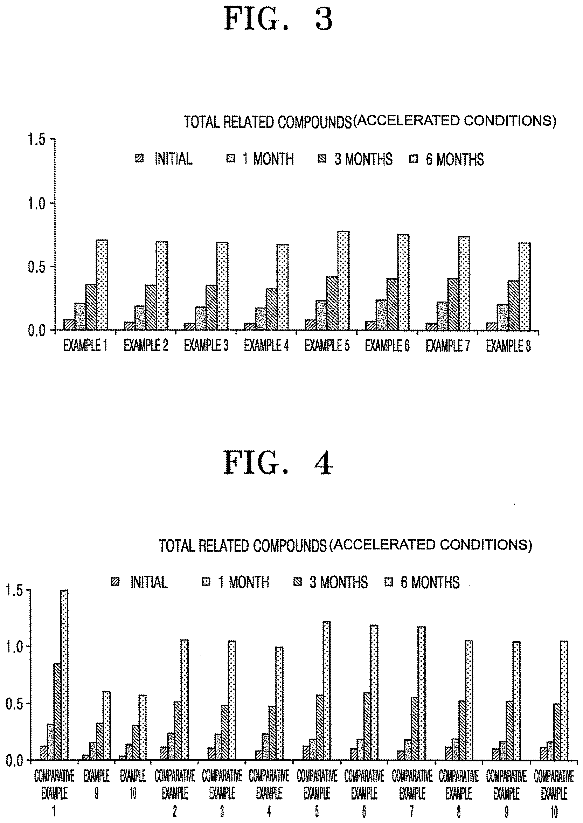

The results of measuring the related compounds of tamsulosin hydrochloride are shown in Tables 4 to 10 and FIGS. 1 to 4.

**[Table 4]**

| Initial | | | | | | | | | | | | |
|---|---|---|---|---|---|---|---|---|---|---|---|---|
| | Example 1 | Example 2 | Example 3 | Example 4 | Example 5 | Example 6 | Example 7 | Example 8 | | | | |
| Total related compounds | 0.08 | 0.06 | 0.05 | 0.05 | 0.08 | 0.07 | 0.05 | 0.06 | | | | |

| | Comparative Example 1 | Example 9 | Example 10 | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 |
|---|---|---|---|---|---|---|---|---|---|---|---|---|
| Total related compounds | 0.12 | 0.04 | 0.03 | 0.11 | 0.10 | 0.08 | 0.12 | 0.10 | 0.08 | 0.11 | 0.10 | 0.11 |

**[Table 5]**

| 1 week under stress conditions | | | | | | | | | | | | |
|---|---|---|---|---|---|---|---|---|---|---|---|---|
| | Example 1 | Example 2 | Example 3 | Example 4 | Example 5 | Example 6 | Example 7 | Example 8 | | | | |
| Total related compounds | 0.12 | 0.11 | 0.11 | 0.10 | 0.15 | 0.14 | 0.14 | 0.14 | | | | |

| | Comparative Example 1 | Example 9 | Example 10 | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 |
|---|---|---|---|---|---|---|---|---|---|---|---|---|
| Total related compounds | 0.25 | 0.11 | 0.08 | 0.17 | 0.16 | 0.16 | 0.18 | 0.18 | 0.18 | 0.19 | 0.16 | 0.16 |

**[Table 6]**

| 2 weeks under stress conditions | | | | | | | | | | | | |
|---|---|---|---|---|---|---|---|---|---|---|---|---|
| | Example 1 | Example 2 | Example 3 | Example 4 | Example 5 | Example 6 | Example 7 | Example 8 | | | | |
| Total related compounds | 0.30 | 0.30 | 0.30 | 0.29 | 0.35 | 0.34 | 0.34 | 0.32 | | | | |

| | Comparative Example 1 | Example 9 | Example 10 | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 |
|---|---|---|---|---|---|---|---|---|---|---|---|---|
| Total related compounds | 0.76 | 0.28 | 0.27 | 0.47 | 0.45 | 0.42 | 0.54 | 0.52 | 0.51 | 0.50 | 0.50 | 0.49 |

**[Table 7]**

| 4 weeks under stress conditions | | | | | | | | | | | | |
|---|---|---|---|---|---|---|---|---|---|---|---|---|
| | Example 1 | Example 2 | Example 3 | Example 4 | Example 5 | Example 6 | Example 7 | Example 8 | | | | |
| Total related compounds | 0.61 | 0.60 | 0.59 | 0.55 | 0.66 | 0.61 | 0.59 | 0.58 | | | | |

| | Comparative Example 1 | Example 9 | Example 10 | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 |
|---|---|---|---|---|---|---|---|---|---|---|---|---|
| Total related compounds | 1.32 | 0.53 | 0.51 | 0.97 | 0.95 | 0.92 | 1.12 | 1.09 | 0.98 | 1.02 | 1.01 | 0.94 |

**[Table 8]**

| 1 month under accelerated conditions | | | | | | | | | | | | |
|---|---|---|---|---|---|---|---|---|---|---|---|---|
| | Example 1 | Example 2 | Example 3 | Example 4 | Example 5 | Example 6 | Example 7 | Example 8 | | | | |
| Total related compounds | 0.21 | 0.19 | 0.18 | 0.18 | 0.24 | 0.24 | 0.23 | 0.21 | | | | |

| | Comparative Example 1 | Example 9 | Example 10 | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 |
|---|---|---|---|---|---|---|---|---|---|---|---|---|
| Total related compounds | 0.31 | 0.15 | 0.13 | 0.23 | 0.22 | 0.22 | 0.18 | 0.18 | 0.18 | 0.19 | 0.16 | 0.16 |

**[Table 9]**

| 3 months under accelerated conditions | | | | | | | | | | | | |
|---|---|---|---|---|---|---|---|---|---|---|---|---|
| | Example 1 | Example 2 | Example 3 | Example 4 | Example 5 | Example 6 | Example 7 | Example 8 | | | | |
| Total related compounds | 0.36 | 0.35 | 0.35 | 0.33 | 0.42 | 0.41 | 0.41 | 0.40 | | | | |

| | Comparative Example 1 | Example 9 | Example 10 | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 |
|---|---|---|---|---|---|---|---|---|---|---|---|---|
| Total related compounds | 0.84 | 0.32 | 0.30 | 0.51 | 0.48 | 0.47 | 0.57 | 0.59 | 0.55 | 0.52 | 0.52 | 0.50 |

**[Table 10]**

| 6 months under accelerated conditions | | | | | | | | | | | | |
|---|---|---|---|---|---|---|---|---|---|---|---|---|
| | Example 1 | Example 2 | Example 3 | Example 4 | Example 5 | Example 6 | Example 7 | Example 8 | | | | |
| Total related compounds | 0.71 | 0.70 | 0.69 | 0.68 | 0.78 | 0.76 | 0.74 | 0.70 | | | | |

| | Comparative Example 1 | Example 9 | Example 10 | Comparative Example 2 | Comparative Example 3 | Comparative Example 4 | Comparative Example 5 | Comparative Example 6 | Comparative Example 7 | Comparative Example 8 | Comparative Example 9 | Comparative Example 10 |
|---|---|---|---|---|---|---|---|---|---|---|---|---|
| Total related compounds | 1.51 | 0.60 | 0.57 | 1.05 | 1.04 | 0.99 | 1.21 | 1.18 | 1.17 | 1.05 | 1.04 | 1.05 |

According to the results of Tables 4 to 10, the total related compounds of Examples 1 to 10 showed good results within the acceptable range, whereas uncoated comparative Example 1 showed an unacceptable result and Comparative Examples 2 to 4 of using the HPMC coating base material showed values which were almost close to the criteria of the total related compounds at 4 weeks after stress conditions and slightly exceeded the acceptable range at 6 months after accelerated conditions. Further, Comparative Examples 5 to 7 of using the HPC coating base material and Comparative Examples 8 to 10 of using ethyl cellulose showed results which exceeded the acceptable range at 4 weeks after stress conditions, except for Comparative Examples 7 and 10 of 6% concentration, and all of them showed unacceptable results at 6 months after accelerated conditions. In conclusion, there was a very great difference in stability between Examples 1 to 10 of using the PVA coating base material and Comparative Examples of using other different coating base materials. Accordingly, it was found that the coating layer including the PVA or PVA-containing copolymer may remarkably improve stabilities of active ingredients, as compared with coating layers including other different kinds of coating base materials.

### Experimental Example 2: Tadalafil Dissolution Test According To Coating Base Material and Amount Thereof

For comparison according to the kind of the coating base material at the same concentration (6%), the capsules of Examples 4 and 8 and Comparative Examples 4, 7, and 10 were subjected to a dissolution test at an initial point, 1 month, 3 months, and 6 months after storage under accelerated conditions (40°C/ 75% RH). Separately, for comparison according to the amount of the PVA coating base material, the capsules of Examples 9 and 10 at an initial point were subjected to a tadalafil dissolution test, and compared with Examples 1 to 4.

A dissolution test was performed using a sinker and 1000 mL of 0.5% SLS solution at 50 rpm according to a dissolution method II (paddle method) of the Korean Pharmacopoeia. Samples were collected at an initial point and 5 min, 10 min, 15 min, 30 min, 45 min, and 60 min after the start of the test, and analyzed by liquid chromatography.
- Column: a column in which a stainless steel tube having an inner diameter of about 4.6 mm and a length of about 5 cm was packed with an octylsilyl silica gel for liquid chromatography having a particle size of about 3.5 µm or a column equivalent thereto (e.g., ZORBAX SB C8 column)
- Detector: UV spectrophotometer (measurement wavelength: 225 nm)
- Flow rate: 2 ml/min
- Injection volume: 50 µl
- Column temperature: 40°C
- Mobile phase: a mixed solution of methanol and purified water at a ratio of 50:50
- QC condition: 0.5 % SLS solution, 1,000 mL

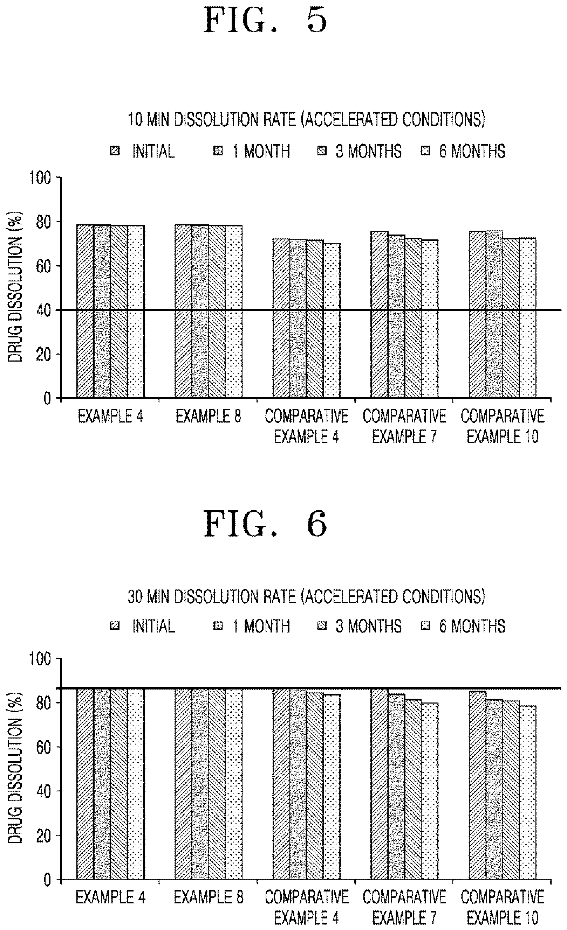

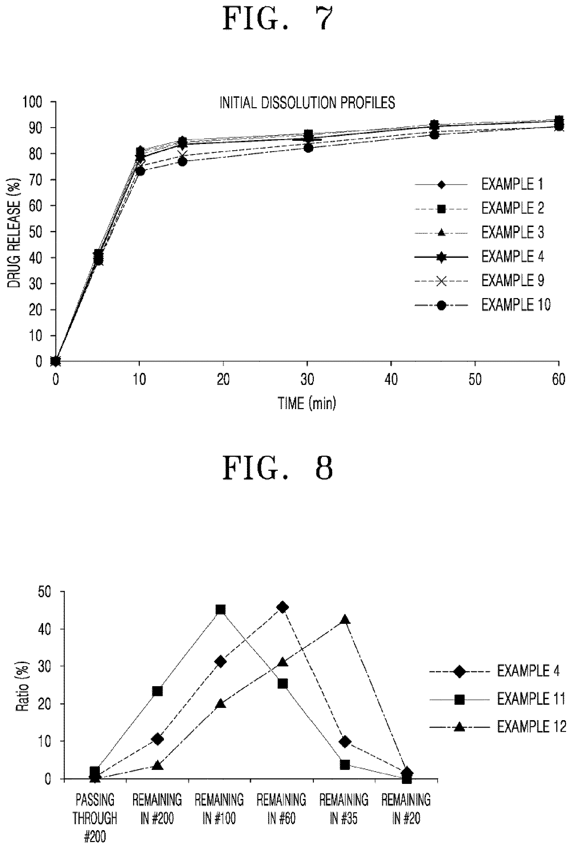

The capsules of Examples 4 and 8 and Comparative Examples 4, 7, and 10 were subjected to a dissolution test at an initial point, 1 month, 3 months, and 6 months after storage under accelerated conditions (40°C/ 75% RH), and tadalafil dissolution results in 10 minutes and 30 minutes are shown in the following Table 11 and FIGS. 5 and 6. Further, dissolution results of Examples 1 to 4 and Examples 9 and 10 at an initial point are shown in the following Table 12 and FIG. 7. A tadalafil dissolution of 40(Q)% or more in 10 minutes and a tadalafil dissolution of 80(Q)% or more in 30 minutes were determined as a reference. Here, definition of Q is as follows. At Stage 1, dissolution of 6 samples is performed, and each sample is determined as acceptable when its Q value exceeds + 5%. When the Q value does not exceed + 5%, additional 6 samples are further tested at Stage 2. When a total of the 12 samples is considered, a mean value of the 12 samples should exceed the Q value, and none of the samples should have the Q value of lower than - 15%. Evan at Stage 2, when the samples were determined as unacceptable, additional 12 samples are further tested. When the 24 samples are considered, a mean value of the samples should exceed the Q value, and the number of samples having the Q value of lower than - 15% should be two or less, and none of the samples should have the Q value of - 25% or lower.

**[Table 11]**

| Dissolution rate | | Accelerated conditions | | | | |
|---|---|---|---|---|---|---|
| 10 min/30 min | | Example 4 | Example 8 | Comparative Example 4 | Comparative Example 7 | Comparative Example 10 |
| Initial | 10 min | 78.52 | 78.38 | 72.22 | 75.36 | 75.26 |
| | 30 min | 86.79 | 86.26 | 86.79 | 86.31 | 84.69 |
| 1 month | 10 min | 78.36 | 78.31 | 71.93 | 73.84 | 75.71 |
| | 30 min | 86.66 | 86.18 | 85.16 | 83.48 | 81.22 |
| 3 month | 10 min | 78.19 | 78.22 | 71.62 | 72.45 | 72.38 |
| | 30 min | 86.24 | 85.97 | 84.26 | 81.34 | 80.64 |
| 6 month | 10 min | 78.16 | 78.13 | 70.29 | 71.66 | 72.45 |
| | 30 min | 85.99 | 85.58 | 83.17 | 79.66 | 78.31 |

**[Table 12]**

| Dissolution rate according to an amount of drug coating | | | | | | | |
|---|---|---|---|---|---|---|---|
| 10 min/30 min | | Example 1 | Example 2 | Example 3 | Example 4 | Example 9 | Example 10 |
| Initial | 10 min | 80.90 | 80.71 | 79.79 | 78.52 | 75.34 | 73.39 |
| | 30 min | 87.48 | 87.54 | 86.99 | 86.79 | 83.63 | 82.53 |

According to Table 11 and FIGS. 5 and 6 which are the results according to the kind of the coating base material, when the PVA coatings of Examples 4 and 8 are compared with other different coating base materials of Comparative Examples 4, 7, and 10, the results of the dissolution tests after 1 month, 3 months, and 6 months under accelerated conditions showed that dissolution tend to decrease at 30 min dissolution rate even at the same concentration. The PVA-coated capsules showed a dissolution decrease of about 1% to about 2%, whereas the HPMC-coated capsules showed a dissolution decrease of about 3% to about 4%, and the ethyl cellulose-coated capsule showed a dissolution decrease of about 5% to about 7%. The tamsulosin related compounds and the tadalafil dissolution test results taken together, it was revealed that, as compared with other coating base materials, the PVA coating may remarkably increase stabilities of the active ingredients without reduction in the initial dissolution rate, and thus it may be used as a coating agent for stability improvement.

Further, according to Table 12 and FIG. 7 which are results of examining the initial tadalafil dissolution rate according to the amount of the PVA coating base material, it was found that when the amount of the coating base material is relatively large, the dissolution rate tend to decrease, and Examples 9 and 10 showed results that do not meet the 30 min dissolution rate criterion due to the high coating agent ratio of 8-10%. Accordingly, when about 1-6% of PVA coating is used, it is possible to obtain a tadalafil initial dissolution pattern that meets the dissolution criterion.

### Experimental Example 3: Tadalafil Dissolution Test According To Granule Size

Granule sizes of wet granules which were prepared during the tadalafil tableting processes of Examples 4, 11 and 12 were measured, and frequency of occurrence of a capping phenomenon at the time of tableting was measured, and a tadalafil initial dissolution rate was measured for the final capsule formulations.

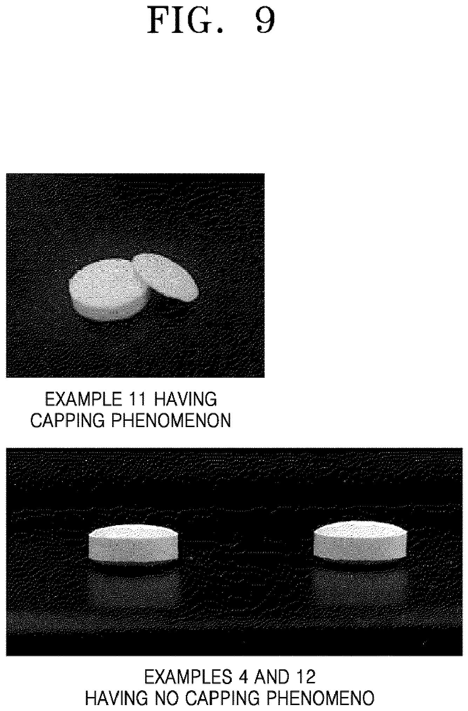

Results of measuring the granule sizes of wet granules which were prepared during the tadalafil tableting processes of Examples 4, 11 and 12 are shown in FIG. 8, and frequency of occurrence of a capping phenomenon at the time of tableting tadalafil tablets, i.e., frequency of occurrence of the capping phenomenon at the time of tableting 100 tablets was examined with the naked eye, and results of measuring the number of capping are shown in Table 13. Photographs of the actual tableted tablets with regard to the presence or absence of the capping phenomenon at the time of tableting the tadalafil tablets of Examples 4, 11, and 12 are shown in FIG. 9.

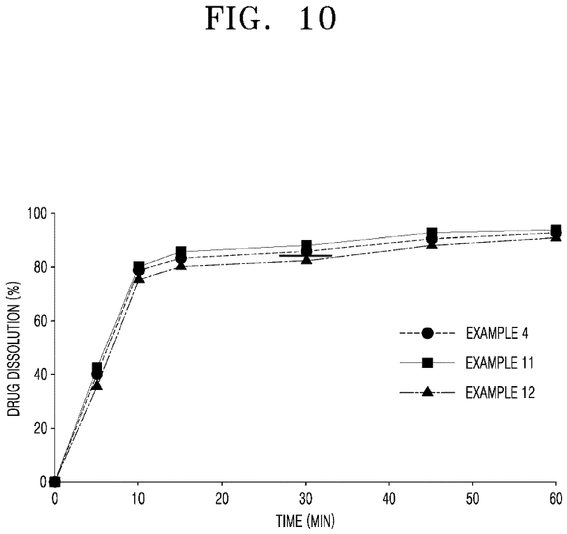

Further, results of measuring the tadalafil dissolution rates of the final capsule formulations are shown in FIG. 10.

**[Table 13]**

| | Example 4 | Example 11 | Example 12 |
|---|---|---|---|
| Number of capping (occurrence/total 100) | 0/100 | 24/100 | 0/100 |

According to the results of FIGS. 8 to 10 and Table 13, Example 11 having a small average particle size showed an acceptable tadalafil initial dissolution rate, but showed the capping phenomenon at the time of tableting, and Example 12 having a large average particle size showed no problem in tableting, but it is highly likely to have an unacceptable tadalafil initial dissolution rate. In contrast to Examples 11 and 12, Example 4 had an appropriate particle size range, and therefore, it showed a reduction in the capping phenomenon at the time of tableting and had an initial dissolution rate that meets the criterion, suggesting that the preferred range of particle size results in increased content uniformity and improved productivity and quality of the formulation.

## Claims

1. A capsule composite formulation comprising, in a separated state, an independent tadalafil part comprising tadalafil or a pharmaceutically acceptable salt thereof; and an independent tamsulosin part comprising tamsulosin or a pharmaceutically acceptable salt thereof, wherein the independent tadalafil part comprises, on a surface thereof, a film coating layer comprising polyvinyl alcohol (PVA) or a PVA-containing copolymer as a coating base material,
wherein the PVA or the PVA-containing copolymer is comprised in an amount of 1% by weight to 6% by weight with respect to the total weight of the independent tadalafil part excluding the coating layer, and
wherein the independent tadalafil part is a tablet of compressed granules, and the granules comprise 10% by weight to 60% by weight of granules having an average granule size of 250 µm to 500 µm and 30% by weight or more of granules having an average granule size of 150 µm or less.

2. The capsule composite formulation of claim 1, wherein the PVA is PVA having a molecular weight of 13,000 to 50,000.

3. The capsule composite formulation of claim 2, wherein the PVA is PVA having a degree of hydrolysis of 86.5% to 89.0%, viscosity of 4.8 mpa.s to 5.8 mpa.s when prepared in a 4 w/w% aqueous solution at about 20°C, and a pH of about 5.0 to about 6.5.

4. The capsule composite formulation of claim 1, wherein the independent tamsulosin part are granules, pellets, tablets, or any combination thereof.

5. The capsule composite formulation of claim 1, comprising
the independent tadalafil part comprising, on a surface thereof, the film coating layer comprising the PVA or PVA-containing copolymer; and an independent tamsulosin part comprising, on a surface thereof, an enteric coating layer comprising an enteric coating base material.

6. The capsule composite formulation of claim 1, wherein the independent tadalafil part is a tablet comprising, on a surface thereof, the film coating layer comprising the PVA or the PVA-containing copolymer, and the independent tamsulosin part is a pellet comprising, on a surface thereof, an enteric coating layer comprising an enteric coating base material.

7. The capsule composite formulation of claim 1, wherein the capsule composite formulation has total related compounds of 1.0% or less which is a criterion determined with reference to ICH Guidelines, as tested based on the impurity test of tamsulosin hydrochloride capsules of the United States Pharmacopoeia (USP), and has a tadalafil dissolution rate of 40(Q)% or more in 10 minutes and a tadalafil dissolution rate of 80(Q)% or more in 30 minutes, according to a dissolution test conducted according to the paddle method of the dissolution test of the USP.

8. The capsule composite formulation of claim 5, wherein the enteric coating base material of the enteric coating layer is selected from the group consisting of a methacrylic acid-ethyl acrylate copolymer, triacetin, shellac, cellulose acetate phthalate, hydroxymethylcellulose phthalate, wax, and any combination thereof.

9. The capsule composite formulation of claim 6, comprising the enteric coating base material in an amount of 0.1% by weight to 20% by weight with respect to the total weight of the independent tamsulosin part.

10. The capsule composite formulation of claim 1, wherein the capsule composite formulation is filled into a hard capsule.

11. The capsule composite formulation of claim 10, wherein a base material of the hard capsule is selected from the group consisting of hypromellose, pullulan, gelatin, polyvinyl alcohol, and any combination thereof.

12. The capsule composite formulation of claim 1, wherein the pharmaceutically acceptable salt of tamsulosin is tamsulosin hydrochloride.

13. The capsule composite formulation of claim 1, wherein the capsule composite formulation is for the prevention or treatment of erectile dysfunction, benign prostatic hyperplasia, or a combination thereof.

14. A method of preparing the capsule composite formulation of any one of claims 1 to 13, the method comprising:
preparing an independent tadalafil part by mixing tadalafil or a pharmaceutically acceptable salt thereof with a pharmaceutically acceptable additive to prepare tadalafil tablet prepared from the granules;
coating the prepared tadalafil tablet with a coating base material comprising PVA or a PVA-containing copolymer;
preparing an independent tamsulosin part by mixing tamsulosin or a pharmaceutically acceptable salt thereof with a pharmaceutically acceptable additive to prepare tamsulosin granules or a pellet or a tablet prepared from the granules; and
encapsulating a hard capsule with the prepared coated tadalafil tablet and the prepared tamsulosin granules, pellet, or tablet, in a separated state.

## Patentansprüche

1. Kapselverbundformulierung, umfassend, in einem separierten Zustand, einen unabhängigen Tadalafil-Teil, umfassend Tadalafil oder ein pharmazeutisch verträgliches Salz davon; und einen unabhängigen Tamsulosin-Teil, umfassend Tamsulosin oder ein pharmazeutisch verträgliches Salz davon, wobei der unabhängige Tadalafil-Teil, auf einer Fläche desselben, eine Filmüberzugsschicht, umfassend Polyvinylalkohol (PVA) oder ein PVA-haltiges Copolymer als Überzugsbasismaterial, umfasst,
wobei der PVA oder das PVA-haltige Copolymer in einer Menge von 1 Gew.-% bis 6 Gew.-% in Bezug auf das Gesamtgewicht des unabhängigen Tadalafil-Teils ausschließlich der Überzugsschicht umfasst ist, und
wobei der unabhängige Tadalafil-Teil eine Tablette aus verpresstem Granulat ist, und das Granulat 10 Gew.-% bis 60 Gew.-% Körnchen mit einer mittleren Körnchengröße von 250 µm bis 500 µm und 30 Gew.-% oder mehr Körnchen mit einer mittleren Körnchengröße von 150 µm oder weniger umfasst.

2. Kapselverbundformulierung nach Anspruch 1, wobei der PVA PVA mit einem Molekulargewicht von 13 000 bis 50 000 ist.

3. Kapselverbundformulierung nach Anspruch 2, wobei der PVA PVA ist mit einem Hydrolysegrad von 86,5% bis 89,0%, einer Viskosität von 4,8 mpa.s bis 5,8 mpa.s, bei Herstellung in einer 4-gewichtsprozentigen wässrigen Lösung bei etwa 20 °C, und einem pH-Wert von etwa 5,0 bis etwa 6,5.

4. Kapselverbundformulierung nach Anspruch 1, wobei der unabhängige Tamsulosin-Teil in Granulat, Pellets, Tabletten oder einer beliebigen Kombination davon besteht.

5. Kapselverbundformulierung nach Anspruch 1, umfassend
den unabhängigen Tadalafil-Teil, umfassend, auf einer Fläche desselben, die Filmüberzugsschicht, umfassend den PVA oder das PVA-haltige Copolymer; und einen unabhängigen Tamsulosin-Teil, umfassend, auf einer Fläche desselben, eine Schicht eines magensaftresistenten Überzugs, umfassend ein Basismaterial des magensaftresistenten Überzugs.

6. Kapselverbundformulierung nach Anspruch 1, wobei der unabhängige Tadalafil-Teil eine Tablette ist, umfassend, auf einer Fläche derselben, die Filmüberzugsschicht, umfassend den PVA oder das PVA-haltige Copolymer, und der unabhängige Tamsulosin-Teil ein Pellet ist, umfassend, auf einer Fläche desselben, eine Schicht eines magensaftresistenten Überzugs, umfassend ein Basismaterial des magensaftresistenten Überzugs.

7. Kapselverbundformulierung nach Anspruch 1, wobei die Kapselverbundformulierung insgesamt 1,0% oder weniger verwandte Verbindungen aufweist, was ein unter Bezugnahme auf ICH-Richtlinien bestimmtes Kriterium ist, wie geprüft auf Grundlage der Unreinheitsprüfung, von Tamsulosin-Hydrochlorid-Kapseln, der US-amerikanischen Pharmakopöe (USP), und eine Tadalafil-Auflösungsrate von 40(Q)% oder mehr in 10 Minuten und eine Tadalafil-Auflösungsrate von 80(Q)% oder mehr in 30 Minuten aufweist, gemäß einer Auflösungsprüfung, die nach dem Blattrührerverfahren der Auflösungsprüfung der USP durchgeführt wird.

8. Kapselverbundformulierung nach Anspruch 5, wobei das Basismaterial des magensaftresistenten Überzugs, der Schicht des magensaftresistenten Überzugs, aus der Gruppe ausgewählt ist, die aus einem Methacrylsäure-Ethylacrylat-Copolymer, Triacetin, Schellack, Celluloseacetatphthalat, Hydroxymethylcellulosephthalat, Wachs und jeder beliebigen Kombination davon besteht.

9. Kapselverbundformulierung nach Anspruch 6, umfassend das Basismaterial des magensaftresistenten Überzugs in einer Menge von 0,1 Gew.-% bis 20 Gew.-% in Bezug auf das Gesamtgewicht des unabhängigen Tamsulosin-Teils.

10. Kapselverbundformulierung nach Anspruch 1, wobei die Kapselverbundformulierung in eine Hartkapsel gefüllt ist.

11. Kapselverbundformulierung nach Anspruch 10, wobei ein Basismaterial der Hartkapsel aus der Gruppe ausgewählt ist, die aus Hypromellose, Pullulan, Gelatine, Polyvinylalkohol und jeder beliebigen Kombination davon besteht.

12. Kapselverbundformulierung nach Anspruch 1, wobei das pharmazeutisch verträgliche Salz von Tamsulosin Tamsulosin-Hydrochlorid ist.

13. Kapselverbundformulierung nach Anspruch 1, wobei die Kapselverbundformulierung der Vorbeugung oder Behandlung von erektiler Dysfunktion, benigner Prostatahyperplasie oder einer Kombination davon dient.

14. Verfahren zur Herstellung der Kapselverbundformulierung nach einem der Ansprüche 1 bis 13, wobei das Verfahren umfasst:
Herstellen eines unabhängigen Tadalafil-Teils durch Mischen von Tadalafil oder einem pharmazeutisch verträglichen Salz davon mit einem pharmazeutisch verträglichen Additiv, um eine aus dem Granulat hergestellte Tadalafil-Tablette herzustellen;
Überziehen der hergestellten Tadalafil-Tablette mit einem Überzugsbasismaterial, welches PVA oder ein PVA-haltiges Copolymer umfasst;
Herstellen eines unabhängigen Tamsulosin-Teils durch Mischen von Tamsulosin oder einem pharmazeutisch verträglichen Salz davon mit einem pharmazeutisch verträglichen Additiv, um Tamsulosin-Granulat oder ein Pellet oder eine Tablette, hergestellt aus dem Granulat, herzustellen; und
Kapselfüllen einer Hartkapsel, mit der hergestellten überzogenen Tadalafil-Tablette und dem/-r hergestellten Tamsulosin-Granulat, Pellet oder Tablette, in einem separierten Zustand.

## Revendications

1. Formulation de capsule composite comprenant, dans un état séparé, une partie indépendante de tadalafil comprenant du tadalafil ou un sel pharmaceutiquement acceptable de celui-ci ; et une partie indépendante de tamsulosine comprenant de la tamsulosine ou un sel pharmaceutiquement acceptable de celle-ci, dans laquelle la partie indépendante de tadalafil comprend, sur une surface de celle-ci, une couche d'enrobage de film comprenant de l'alcool polyvinylique (PVA) ou un copolymère contenant du PVA en tant que matériau de base d'enrobage,
dans laquelle le PVA ou le copolymère contenant du PVA est compris dans une quantité de 1 % en poids à 6 % en poids par rapport au poids total de la partie indépendante de tadalafil à l'exclusion de la couche d'enrobage, et
dans laquelle la partie indépendante de tadalafil est un cachet de granulés comprimés et les granulés comprennent de 10 % en poids à 60 % en poids de granulés ayant une taille de granulés moyenne de 250 µm à 500 µm et 30 % en poids ou plus de granulés ayant une taille de granulés moyenne de 150 µm ou moins.

2. Formulation de capsule composite selon la revendication 1, dans laquelle le PVA est un PVA ayant un poids moléculaire de 13.000 à 50.000.

3. Formulation de capsule composite selon la revendication 2, dans laquelle le PVA est un PVA ayant un degré d'hydrolyse de 86,5 % à 89,0 %, une viscosité de 4,8 mpa.s à 5,8 mpa.s lorsqu'il est préparé dans une solution aqueuse à 4 % p/p à environ 20°C, et un pH d'environ 5,0 à environ 6,5.

4. Formulation de capsule composite selon la revendication 1, dans laquelle la partie indépendante de tamsulosine est des granulés, des pastilles, des cachets ou toute combinaison de ceux-ci.

5. Formulation de capsule composite selon la revendication 1, comprenant :
la partie indépendante de tadalafil comprenant, sur une surface de celle-ci, la couche d'enrobage de film comprenant le PVA ou le copolymère contenant du PVA ; et une partie indépendante de tamsulosine comprenant, sur une surface de celle-ci, une couche d'enrobage entérique comprenant un matériau de base d'enrobage entérique.

6. Formulation de capsule composite selon la revendication 1, dans laquelle la partie indépendante de tadalafil est un cachet comprenant, sur une surface de celui-ci, la couche d'enrobage de film comprenant le PVA ou le copolymère contenant du PVA ; et la partie indépendante de tamsulosine est une pastille comprenant, sur une surface de celle-ci, une couche d'enrobage entérique comprenant un matériau de base d'enrobage entérique.

7. Formulation de capsule composite selon la revendication 1, où la formulation de capsule composite a un total de composés apparentés de 1,0 % ou moins qui est un critère déterminé en faisant référence aux directives ICH, comme il est testé sur la base de l'essai d'impuretés de capsules de chlorhydrate de tamsulosine de la pharmacopée des Etats-Unis (USP) et a une vitesse de dissolution de tadalafil de 40(Q) % ou plus en 10 minutes et une vitesse de dissolution de tadalafil de 80(Q) % ou plus en 30 minutes, selon un essai de dissolution mené selon la méthode utilisant un appareil à palette de l'essai de dissolution de l'USP.

8. Formulation de capsule composite selon la revendication 5, dans laquelle le matériau de base d'enrobage entérique de la couche d'enrobage entérique est choisi dans le groupe constitué d'un copolymère d'acide méthacrylique-acrylate d'éthyle, de triacétine, de gomme laque, de cellulose acétate phtalate, d'hydroxyméthylcellulose phtalate, d'une cire et de toute combinaison de ceux-ci.

9. Formulation de capsule composite selon la revendication 6, comprenant le matériau de base d'enrobage entérique dans une quantité de 0,1 % en poids à 20 % en poids par rapport au poids total de la partie indépendante de tamsulosine.

10. Formulation de capsule composite selon la revendication 1, où la formulation de capsule composite est remplie dans une capsule dure.

11. Formulation de capsule composite selon la revendication 10, dans laquelle un matériau de base de la capsule dure est choisi dans le groupe constitué d'hypromellose, de pullulane, de gélatine, d'alcool polyvinylique et de toute combinaison de ceux-ci.

12. Formulation de capsule composite selon la revendication 1, dans laquelle le sel pharmaceutiquement acceptable de tamsulosine est le chlorhydrate de tamsulosine.

13. Formulation de capsule composite selon la revendication 1, où la formulation de capsule composite est pour la prévention ou le traitement d'un dysfonctionnement érectile, d'une hyperplasie prostatique bénigne ou d'une combinaison de ceux-ci.

14. Procédé de préparation de la formulation de capsule composite selon l'une quelconque des revendications 1 à 13, le procédé comprenant :
la préparation d'une partie indépendante de tadalafil par le mélange de tadalafil ou d'un sel pharmaceutiquement acceptable de celui-ci avec un additif pharmaceutiquement acceptable pour préparer un cachet de tadalafil préparé à partir des granulés ;
l'enrobage du cachet de tadalafil préparé avec un matériau de base d'enrobage comprenant du PVA ou un copolymère contenant du PVA ;
la préparation d'une partie indépendante de tamsulosine par le mélange de tamsulosine ou d'un sel pharmaceutiquement acceptable de celle-ci avec un additif pharmaceutiquement acceptable pour préparer des granulés de tamsulosine ou une pastille ou un cachet préparé à partir des granulés ; et
l'encapsulation d'une capsule dure avec le cachet de tadalafil enrobé préparé et les granulés, la pastille ou le cachet de tamsulosine préparés, dans un état séparé.
